# EUROPEAN PATENT APPLICATION

(11) **EP 4 789 553 A1**
(43) Date of publication of application: **12.08.2026**
(21) Application number: 25156347.4
(22) Date of filing: 06.02.2025
(51) Int. Cl.: A24B 13/00, A24B 15/16, A24B 15/30, A24B 15/42, A61K 31/465

(54) **A POUCHED NICOTINE PRODUCT FOR ORAL USE COMPRISING A PH STABILIZING AGENT**

(71) Applicant: Swedish Match North Europe AB, 118 85 Stockholm (SE)
(72) Inventor: Hosseini, Seyedehsan, 413 26 Göteborg (SE)
(74) Representative: Valea AB

(57) **Abstract**

The present disclosure relates to an oral pouched nicotine product comprising a filling material and a saliva-permeable pouch enclosing the filling material, the filling material comprising
- a nicotine compound;
- a pH-regulating agent; and
- a monovalent metal salt of a stearic acid.

## Description

### TECHNICAL FIELD

The present disclosure relates to a pouched product for oral use comprising a liquid permeable cover material and a portion sized amount of a filling material comprising a combination of a pH regulating agent and a pH stabilizing agent acid to improve the stability of pH in the filling material when said oral pouched nicotine product is stored.

### BACKGROUND

Oral smokeless products, i.e. smokeless products for oral use, are well known in the art and include tobacco products as well as non-tobacco products.

The oral smokeless products are typically provided as pouched products being portion-packed in a saliva-permeable, porous wrapper material forming a pouch enclosing a filling material. The pouch material used in pouched products for oral use, also called the packaging material, is commonly a nonwoven material, such as a dry-laid chemically bonded nonwoven comprising regenerated cellulose fibres, e.g., viscose fibres. The oral smokeless product is typically used by a consumer by placing it between the upper or lower gum and the lip and retaining it there for a limited period of time.

Unprotonated nicotine (free-base nicotine) is inherently unstable and susceptible to oxidation when exposed to air. In acidic conditions at a pH of approximately 4.5-5.5 nicotine transitions into its mono-protonated and di-protonated forms, which are suitable for storage (due to their resistance to oxidation). Although free-base nicotine is unstable, it is the form most efficiently absorbed by the human mucosa. Consequently, nicotine should be stored under acidic conditions but converted to its non-protonated free base form for use.

To increase the bioavailability of nicotine in smokeless tobacco and non-tobacco products for the user, a pH regulating agent is added to the filling material to raise the pH of the product to somewhere between 7.5 and 9.

However, despite the addition of a pH regulating agent such as carbonates or bicarbonates, the pH of oral smokeless tobacco free products has been found to be unstable during storage.

An objective with the oral pouched nicotine product disclosed herein is to offer an oral pouched product containing a filling material having improved shelf-life during storage of these products, in particular with regards to the stability of pH.

### SUMMARY

The above object may be achieved with an oral pouched product according to claim 1. Variations of the disclosure are set out in the dependent claims and in the following description.

According to a first aspect of the disclosure, there is provided an oral pouched nicotine product comprising a filling material and a saliva-permeable pouch enclosing the filling material, the filling material comprising a nicotine compound, a pH-regulating agent and a monovalent metal salt of a stearic acid.

The first aspect of the disclosure may seek to provide an oral pouched nicotine product wherein the pH of the filling material remains stable during storage of the oral pouched nicotine product. A technical benefit may include that a stable pH helps prevent fluctuations in the flavor profile of the product, as well as the bioavailability of nicotine for the consumer. Certain flavorants present in the filling material may lose their taste and may even attain a foul taste if the pH of the filling material becomes too alkaline. In contrast, the uptake of nicotine by the mucosa in the oral cavity of the user may decrease if the pH decreases too much towards a more acidic level. It is therefore of utmost importance to maintain the pH of the filling material in the oral pouched nicotine product during storage until the product is consumed by the user.

Optionally in some examples, including at least one preferred example, the monovalent metal salt of a stearic acid is selected from the group consisting of sodium stearate, potassium stearate and a combination thereof. A technical benefit may include that inclusion of sodium stearate and/or potassium stearate in the filling material of the oral pouched nicotine products stabilizes the pH of the products significantly compared to filling material without any addition of stearate. The same stabilizing effect on pH cannot be seen from inclusion of divalent metal salts of stearic acids such as e.g., magnesium stearate or calcium stearate. Advantageously, the monovalent metal salt of a stearic acid is sodium stearate.

Optionally in some examples, including at least one preferred example, the monovalent metal salt of a stearic acid is present in an amount of less than 20 wt.-% based on the total weight of the filling material, such as from about 0.1 wt.-% to about 20 wt.-% based on the total weight of the filling material. A technical benefit may include that already at a low concentration of the monovalent metal salt of a stearic acid, the pH of the filling material becomes significantly more stable than when sodium stearate is not present.

Optionally in some examples, including at least one preferred example, the pH regulating agent is present in an amount within the range of from about 0.02 wt.-% to about 20 wt.-%, such as from about 0.04 wt.-% to about 0.15 wt.-% or from about 2 wt.-% to about 5 wt.-%, or from about 6 wt.-% to about 10 wt.-%, or from about 11 wt.-% to about 15 wt.-%, or from about 12 wt.-% to about 20 wt.-% based on the total weight of the filling material. A technical benefit of adding a pH regulating agent to a filling material may include that the pH of the filling material is raised from an acidic pH where nicotine is in its stable form and may be stored since it is less prone to oxidation, to an alkaline pH where nicotine becomes more available for uptake by the mucosa of the user. The presence of a monovalent metal salt of a stearic acid in the filling material helps maintain the pH of the oral pouched nicotine products at a stable level until it reaches the consumer.

Optionally in some examples, including at least one preferred example, the pH regulating agent is a carbonate or a bicarbonate, and is selected from the group consisting of sodium carbonate, potassium carbonate, sodium bicarbonate, potassium bicarbonate and any combination thereof. Preferably the pH regulating agent included in the oral pouched nicotine product is sodium carbonate or sodium bicarbonate. A technical benefit of selecting one of the listed carbonates and bicarbonates in this group may include that all four compounds are readily available and commonly used in the food industry since they are considered safe to consume when used appropriately and within recommended amounts.

Optionally in some examples, including at least one preferred example, the filling material does not comprise a tobacco material. A technical benefit may include that an oral pouched non-tobacco nicotine product is provided.

Optionally in some examples, including at least one preferred example, the filling material may comprise a tobacco material in an amount within the range of from about 0.1 wt.-% to about 5 wt.-%, such as from about 0.1 wt.-% to about 10 wt.-%, such as from about 0.1 wt.-% to about 5 wt.-%, such as from about 0.1 wt.-% to about 1 wt.-%, based on the total weight of the filling material. A technical benefit may include that an oral pouched low-tobacco nicotine product is provided. The presence of this small amount of tobacco will not impact the pH of the product to be substantially different from that exhibited by the oral pouched tobacco free products described herein.

Optionally in some examples, including at least one preferred example, the pouched nicotine product has a moisture content in the range of from about 1.0 wt.-% to about 60 wt.-%, such as from about 20 wt.-% to about 60 wt.-% such as from about 1 wt.-% to about 20 wt.-%, based on the total weight of the filling material. A technical benefit may include that the oral pouched nicotine product can be provided either as a moist oral pouched nicotine product with a moisture content from about 20 wt.-% or above, and/or as a dry product with a moisture content from 20 wt.-% or less.

Optionally in some examples, including at least one preferred example, the filling material of said oral pouched nicotine product has a pH from about 5 to about 10, such as from about 7 to about 9.5, such as from about 8 to about 9, when the filling material is dispersed in water. A technical benefit may include that at a pH within this range provides an oral pouched nicotine product with a good flavor and optimal bioavailability of nicotine for the consumer.

Optionally in some examples, including at least one preferred example, at least part of the filling material is water insoluble. A technical benefit may include that a water insoluble material does not dissolve when subjected to saliva in the oral cavity of a user and retains or substantially retains its shape when incorporated in a pouched nicotine product for oral use.

Optionally in some examples, including at least one preferred example, the filling material comprises one or more of maize fibers, oat fibers, tomato fibers, barley fibers, rye fibers, sugar beet fibers, buck wheat fibers, wheat fibers, pea fibers, potato fibers, apple fibers, cocoa fibers, bamboo fibers, and citrus fibers. A technical benefit may include that the above listed botanical non-tobacco materials are mostly water insoluble and will not dissolve when subjected to saliva in the oral cavity of a user and thereby retain or substantially retain its shape when incorporated in the filling material of the oral pouched nicotine product.

Optionally in some examples, including at least one preferred example, the filling material comprises microcrystalline cellulose. A technical benefit of incorporating microcrystalline cellulose in the filling material may be that microcrystalline cellulose is a chemically inert material and will not dissolve when subjected to saliva in the oral cavity of a user.

Optionally in some examples, including at least one preferred example, the nicotine compound is selected from the group consisting of nicotine hydrochloride, nicotine dihydrochloride, nicotine monotartrate, nicotine bitartrate, nicotine bitartrate dihydrate, nicotine sulphate, nicotine zinc chloride monohydrate, nicotine salicylate, nicotine polacrilex and any combination thereof. A technical benefit may include that the above listed nicotine compounds are nicotine salts formed by reacting nicotine with acids. Nicotine salts are more stable than nicotine in its free base which is prone to oxidation.

Optionally in some examples, including at least one preferred example, the nicotine compound is one or more of nicotine bitartrate and nicotine bitartrate dihydrate. A technical benefit may include that nicotine bitartrate and nicotine bitartrate dihydrate are nicotine salts that tend to be less harsh on the oral mucosa, making the oral pouched nicotine product more comfortable to use. These salts may help in reducing the bitterness associated with nicotine thereby improving the overall tase of the product. Nicotine bitartrate enhances the absorption efficiency of nicotine, ensuring a more consistent and effective delivery. Nicotine bitartrate dihydrate is chemically stable, which helps maintain the efficacy of the oral pouched nicotine product over time.

Optionally in some examples, including at least one preferred example, the pH of said filling material of said oral pouched nicotine product does not exceed 9.5 upon storage and/or changes by no more than plus or minus 0.5 pH units upon storage, such as storage taking place at a relative humidity from 60 percent to 75 percent, at a temperature from 22 degrees centigrade to 30 degrees centigrade, and/or for a time of 15 weeks. A technical benefit of incorporating a monovalent metal salt of a stearic acid in the filling material may include that the pH of the oral pouched nicotine product remains stable during storage at a relative humidity from 60 percent to 75 percent, at a temperature from 22 degrees centigrade to 30 degrees centigrade and/or for as long as 15 weeks.

According to a second aspect of the disclosure, there is provided a packaging container comprising a plurality of oral pouched nicotine products as described herein.

According to a third aspect of the disclosure, there is provided a method for manufacturing a filling material as described herein.

The disclosed aspects, examples (including any preferred examples), and/or accompanying claims may be suitably combined with each other as would be apparent to anyone of ordinary skill in the art. Additional features and advantages are disclosed in the following description, claims, and drawings, and in part will be readily apparent therefrom to those skilled in the art or recognized by practicing the disclosure as described herein.

### BRIEF DESCRIPTION OF THE DRAWINGS

The present invention will be further explained hereinafter by means of non-limiting examples and with reference to the appended drawings wherein:
Figures 1A and 1B show effects on pH from storing pouched nicotine products containing 0.9 wt.-% of nicotine and stearate in zone II.....
Figures 2A and 2B show effects on pH from storing pouched nicotine products containing 1.6 wt.-% of nicotine and stearate in zone II.
Figures 3A and 3B show effects on pH from storing pouched nicotine products containing 0.9 wt.-% of nicotine and stearate in zone IV.
Figures 4A and 4B show effects on pH from storing pouched nicotine products containing 1.6 wt.-% of nicotine and stearate in zone IV.
Figures 5A and 5B disclose the effects on pH of high concentration of sodium stearate during storage of pouched nicotine products in zone II.
Figures 6A and 6B disclose the effects on pH of high concentration of sodium stearate during storage of pouched nicotine products in zone IV.

### DETAILED DESCRIPTION

The oral pouched nicotine product described herein comprises a filling material and a saliva-permeable pouch of a packaging material enclosing the filling material. The filling material comprises a nicotine containing material, a pH-regulating agent and a monovalent metal salt of stearic acid.

The terms *"oral"* and *"oral use"* refer to a use of a product in contact with mucous membranes in the oral cavity of a human being, such as buccal placement of the product in the oral cavity. The products for oral use as disclosed herein are intended to be placed in their entirety in the oral cavity and are not intended to be swallowed.

As used herein the terms *"pouched product for oral use"* or *"oral pouched product"* refer to a portion of a smokeless composition containing saliva extractables and being packed in a saliva-permeable pouch material.

By a *"cover material"* as used herein is implied any suitable saliva permeable packaging material as known in the art. The cover material may also be referred to as *"pouch material"* and may be a nonwoven material, a material made by conventional textile production methods such as weaving or knitting or may be an apertured plastic film or netting. A nonwoven material suitable for use as cover material may be a nonwoven material comprising staple fibres, such as staple fibres of regenerated cellulose e.g., viscose rayon staple fibres and a binder, such as a polyacrylate binder. Such nonwoven materials are commonly produced by carding the staple fibres to form a fibrous web, followed by consolidating the carded fibrous web by means of the binder. Alternatively, the nonwoven material may comprise fibres which are formed into a nonwoven web by spunbonding, hydroentangling, meltblowing, etc. The fibres used in such processes are generally thermoplastic fibres which are thermally bonded to form a coherent nonwoven web.

Commonly used cover materials, such as the viscose nonwovens disclosed herein, are generally inelastic and non-stretchable materials which are relatively inexpensive to produce and easy to handle in a production process.

Pouched products for oral use are normally sized and configured to fit comfortably and discreetly in a user's mouth between the upper or lower gum and the lip. In general, pouched products for oral use have a generally rectangular shape, although other shapes are possible. Some typical shapes (length x width) of commercially available pouched products for oral use are, for instance, 35 mm x 20 mm, 34/35 mm x 14 mm, 33/34 mm x 18 mm, 27/28 mm x 14 mm, 34 mm x 10 mm and 38 x 14 mm. Typical pouched products for oral use may have a maximum length within the range of from 15 mm to 80 mm along the longitudinal direction of the product and a maximum width within the range of from 5 mm to 50 mm along the transverse direction of the product. The pre-use thickness of the pouched product is normally within the range of from 1 mm to 20 mm. The total weight of commercially available pouched products for oral use is typically within the range from about 0.1 g to about 5 g, such as from about 0.4 g to 1.8 g, per pouched product.

A "user container" typically contains in the range of 10-30 pouched products, such as in the range of 20 to 25 pouched products. The pouched products may be placed randomly in the user container or in a pattern, for instance as described in WO 2012/069505 A1. The user container as disclosed herein is a consumer package having a shape and a size adapted for conveniently carrying the consumer package in a pocket or in a handbag and may be used for packaging any known type of pouched product for oral use. The user container may include a disposal compartment for storage of used oral pouched products.

The disposal compartment is separated from the compartment in the container where the fresh oral pouched products are stored until use.

As used herein, the term *"moisture content"* refers to the percent by weight, wt.-%, of oven volatile substances, such as water and other oven volatiles which is present in a component material, a composition or a product and is determined according to the Loss on Drying (LOD) method disclosed herein.

The *"dry weight"* of a material, a composition, or a product is calculated by detracting the amount of moisture from the total weight of the material, composition or product, the moisture content being determined by the Loss on Drying method as disclosed herein.

The term "filling material" as used herein refers to the material enclosed by the pouch material such as e.g., water-insoluble and water-soluble materials, non-tobacco or tobacco material, natural fibers, nicotine compounds, pH regulating agents, metal salts of stearic acids, humectants, sweeteners, flavorants, salts and water/moisture.

The filling material of the oral pouched nicotine product described herein may comprise non-tobacco material which may be water-insoluble, water-soluble or a combination thereof. Water-insoluble non-tobacco material does not dissolve when subjected to saliva in the oral cavity of a user and retains or substantially retains its shape when incorporated in a pouched product for oral use.

The non-tobacco material as described herein may be selected from the group consisting of cellulose such as microcrystalline cellulose, powdered cellulose, spherical cellulose and a combination thereof.

Additionally, or alternatively, the non-tobacco material of the oral pouched nicotine product described herein may also comprise one or more of maize fibers, oat fibers, tomato fibers, barley fibers, rye fibers, sugar beet fibers, buck wheat fibers, wheat fibers, pea fibers, potato fibers, apple fibers, cocoa fibers, bamboo fibers, citrus fibers, and any combination thereof. In an example, the fibers may form part of the non-tobacco material.

Advantageously the water insoluble non-tobacco material may comprise or consist of cellulose such as microcrystalline cellulose and/or powdered cellulose, or spherical cellulose or bamboo fiber. For instance, the filling material may comprise microcrystalline cellulose.

The non-tobacco material of the oral pouched nicotine product described herein may further comprise a water-soluble material such as a sugar alcohol selected from the group consisting of maltitol, mannitol, xylitol, sorbitol, or a combination thereof.

The filling material of the oral pouched nicotine product as described herein may also comprise particles having a diameter from 0.1 mm to 2.0 mm. In one example the particles may comprise a nicotine source and are formed from a carrier material comprising microcrystalline cellulose. In another example the particles may have a core with an outer surface, wherein the core comprises a material selected from the group consisting of cellulose, starch, polyalcohol, sugar and any combinations thereof. A first coating which is applied on at least part of the outer surface, comprises a binder and nicotine. In both examples low amounts of tobacco may optionally be included in the particles.

The oral pouched nicotine product may be free from tobacco, i.e. an oral pouched nicotine non-tobacco product.

Alternatively, the oral pouched nicotine product may comprise a low amount of tobacco material thereby providing an oral pouched low tobacco nicotine product.

The term "tobacco material" is used herein for tobacco leaves or parts of leaves, such as lamina and stem of any member of the genus Nicotiana. The leaves and parts of leaves may be finely divided (disintegrated), such as ground, cut, shredded, threshed, granulated and the parts of leaves may be blended in defined proportions in the tobacco material.

The amount of tobacco material of the oral pouched low tobacco nicotine product may be within the range of from about 0.05 percent to about 20 percent by weight such as from about 0.1 percent to about 10 percent by weight, such as from about 0.1 wt.-% to about 5 wt.-%, such as from about 0.1 wt.-% to about 2 wt.-%, based on the total weight of the filling material. The presence of this small amount of tobacco will not impact the pH of the product to be substantially different from that exhibited by the oral pouched tobacco free products described herein.

The tobacco material may be provided in a form as described herein.

Further, the tobacco material may be a purified tobacco material, such as a bleached tobacco material or a tobacco extract.

The tobacco material described herein may comprise one, two or more non-tobacco materials as listed herein.

The filling material may comprise one, two or more nicotine compounds.

The nicotine compound may be but is not limited to a nicotine salt and/or nicotine base, free nicotine, protonated nicotine, a nicotine salt complex or a combination thereof. The nicotine compound such as nicotine base may be bound to an ion exchange resin, such as polacrilex, e.g. via a salt bridge. Alternatively, or additionally, the ion exchange resin may function as a solid support for the nicotine compound such as nicotine base.

Nicotine base, such as in the form of an oily liquid, may be synthetically produced or extracted from tobacco.

The nicotine compound may be a nicotine salt such as a nicotine salt selected from the group consisting of nicotine hydrochloride, nicotine dihydrochloride, nicotine monotartrate, nicotine bitartrate, nicotine bitartrate dihydrate, nicotine sulphate, nicotine zinc chloride monohydrate, nicotine salicylate, nicotine citrate, nicotine polacrilex and any combination thereof. In particular, the filling material may comprise nicotine bitartrate and/or nicotine bitartrate di hydrate.

The filling material of the oral pouched nicotine product as described herein comprises within the range of from about 0.1 wt.- to about 30 wt.- of the nicotine compound, based on the total weight of the filling material, such as from about 0.2 wt.-% to about 25 wt.-%, such as from about 0.3 wt.-% to about 20 wt.-%, such as from about 0.4 wt.-% to about 15 wt.-%, such as from about 0.5 wt.-% to about 10 wt.-% of nicotine, based on the total weight of the filling material.

The amount of nicotine compound such as nicotine salt and/or nicotine base per pouched product may be within the range from about 0.1 mg to about 20 mg of nicotine calculated as nicotine base, such as about 0.5 mg, about 1.0 mg, about 1.5 mg, about 2.0 mg, about 2.5 mg, about 3.0 mg, about 3.5 mg, about 4.0 mg, about 4.5 mg, about 5.0 mg, about 6.0 mg, about 7.0 mg, about 8.0 mg, about 9.0 mg, about 10 mg, about 12 mg, about 14 mg, about 16 mg, about 18 mg, or about 20 mg of nicotine per pouched product.

The nicotine salt of the filling material in the oral pouched nicotine product as disclosed herein may be a nicotine salt present in solid form and/or dissolved form.

The nicotine compound as disclosed herein may be adsorbed or non-adsorbed onto the non-tobacco material as disclosed herein. It will be appreciated that the expression "adsorbed onto" means that the nicotine compound adheres to an outer surface of the non-tobacco material. When the nicotine compound is adsorbed onto the non-tobacco material it adheres to the outer surface of said non-tobacco material without substantially penetrating into any void(s) of said non-tobacco material.

Unprotonated nicotine (free-base nicotine) is inherently unstable and susceptible to oxidation when exposed to air. In acidic conditions at a pH of approximately 4.5-5.5 nicotine transitions into its mono-protonated and di-protonated forms, which are suitable for storage (due to their resistance to oxidation). Although free-base nicotine is unstable, it is the form most efficiently absorbed by the human mucosa. Consequently, nicotine should be stored under acidic conditions but converted to its non-protonated free base form for use.

To increase the availability of free base nicotine and improve the absorption efficiency of nicotine by the user, a pH regulating agent is added to the filling material. A "pH regulating agent" as used herein, is one or more substances added with the purpose of adjusting the pH of an oral pouched nicotine product.

The pH regulating agent is selected from the group consisting of Na₂CO₃, K₂CO₃, NaHCOs, KHCO₃ and a combination thereof is added to the filling material.

The pH regulating agent of the oral pouched nicotine product as described herein allows the pH of the oral pouched nicotine product to be within the range of from about 5 to about 10, such as from about 7 to about 9.5, such as from about 7 to about 9.2, such as from about 7 to about 9, such as from about 8 to about 9. This pH may be achieved during or after manufacture of the product, such as immediately after manufacture of the product.

To achieve and/or maintain a pH of the filling material within the desired range of about 5 to 10, a pH regulating agent is present in the filling material of the oral pouched nicotine product in an amount within the range of about 0.02 wt.-% to about 20 wt.-%, such as from about 0.1 wt.-% or more, such as from about 0.2 wt.-% or more, such as from about 0.3 wt.-% or more, such as from about 0.4 wt.-% or more, such as from about 0.5 wt.-% or more, such as from about 0.6 wt.-% or more, such as from about 0.7 wt.-% or more, such as from about 0.8 wt.-% or more, such as from about 0.9 wt.-% or more, based on the total weight of the filling material of the oral pouched nicotine product. The pH regulating agent is present in the filling material of the oral pouched nicotine product in an amount from about 9 wt.-% or less, such as from about 8 wt.-% or less, such as from about 7 wt.-% or less, such as from about 6 wt.-% or less, such as from about 5 wt.-% or less based on the total weight of the filling material of the oral pouched nicotine product.

Advantageously the pH regulating agent is added in an amount from about 0.04 wt.-% to about 0.15 wt.-%, or from about 0.2 wt.-% to about 3 wt.-%, or from about 2wt.-% to about 5 wt.-%, or from about 6 wt.-% to about 10 wt.%, or from about 11 wt.% to about 15 wt.-% or from about 12 wt.-% to about 20 wt.-% based on the total weight of the filling material of the oral pouched nicotine product.

Due to different reactions occurring in the filling material during storage, the pH of the pouched product may be affected and increase or decrease over time.

To remedy the instability of pH in the oral pouched nicotine products comprising Na₂CO₃, K₂CO₃, NaHCOs and/or KHCO₃ as pH regulating agent during storage, the present inventors have found that an addition of a monovalent metal salt of a stearic acid as described below to the filling material, counteracts this instability in pH during storage of the oral pouched nicotine products.

The monovalent metal salts of a stearic acid described herein consist of sodium stearate or potassium stearate, or a combination thereof. Sodium stearate (IUPAC: Sodium Octadecanoate) with the chemical formula C₁₈H₃₅NaO₂ is the sodium salt of stearic acid.

Potassium stearate (IUPAC: Potassium Octadecanoate) with the chemical formula C₁₈H₃₅KO₂ is the potassium salt of stearic acid.

The monovalent metal salt of a stearic acid described herein may be present in the filling material of the pouched product in an amount from about 0.1 wt.-% to about 20 wt.-% based on the total amount of the filling material, such as from about 0.1 wt.-% or more, such as from about 0.2 wt.-% or more, such as from about 0.3 wt.-% or more, such as from about 0.4 wt.-% or more, such as from about 0.5 wt.-% or more, such as from about 0.6 wt.-% or more, such as from about 0.7 wt.-% or more, such as from about 0.8 wt.-% or more, such as from about 0.9 wt.-% or more, such as from about 1.0 wt.-% or more based on the total amount of the filling material.

The monovalent metal salt of a stearic acid described herein may be present in the filling material of the pouched product in an amount from about 15 wt.-% or less, such as from about 10 wt.-% or less, such as from about 9 wt.-% or less, such as from about 8 wt.-% or less, such as from about 7 wt.-% or less, such as from about 6 wt.-% or less, such as from about 5 wt.-% or less, based on the total amount of the filling material.

Advantageously the monovalent metal salt of stearic acid is added in amount of from about 0.3 wt.-% to about 0.6 wt.-%, or from about 0.4 wt.-% to about 1 wt.-%, or from about 0.6 wt.-% to about 2 wt.-% based on the total amount of the filling material. It has been found that a combination of a pH regulating agent as described herein and a monovalent metal salt of a stearic acid stabilizes the pH of the filling material during storage of the oral pouched nicotine product.

Surprisingly, the same pH stabilizing effect was not observed for divalent metal salts of stearic acid such as e.g., magnesium stearate or calcium stearate as can be seen in the experiments disclosed below.

In one example the oral pouched nicotine product comprises a filling material comprising nicotine, sodium stearate, sodium carbonate and/or sodium bicarbonate.

In one example the oral pouched nicotine product comprises a filling material comprising nicotine, sodium stearate, potassium carbonate and/or potassium bicarbonate.

In one example the oral pouched nicotine product comprises a filling material comprising nicotine, sodium stearate, sodium carbonate and potassium carbonate.

In one example the oral pouched nicotine product comprises a filling material comprising nicotine, sodium stearate, sodium carbonate and potassium bicarbonate.

In one example the oral pouched nicotine product comprises a filling material comprising nicotine, sodium stearate, sodium bicarbonate and potassium bicarbonate.

In one example the oral pouched nicotine product comprises a filling material comprising nicotine, potassium stearate, potassium carbonate and/or potassium bicarbonate.

In one example the oral pouched nicotine product comprises a filling material comprising nicotine, potassium stearate, sodium carbonate and/or sodium bicarbonate.

In one example the oral pouched nicotine product comprises a filling material comprising nicotine, potassium stearate, sodium carbonate and potassium carbonate.

In one example the oral pouched nicotine product comprises a filling material comprising nicotine, potassium stearate, sodium carbonate and potassium bicarbonate.

In one example the oral pouched nicotine product comprises a filling material comprising nicotine, potassium stearate, sodium bicarbonate and potassium bicarbonate.

In a preferred embodiment the monovalent metal salt is sodium stearate.

The oral pouched nicotine product may be dry or moist. The storage may be as described herein.

The filling material of the oral pouched nicotine product may further comprise a sweetener such as e.g., Acesulfame potassium, xylitol or sorbitol, maltitol.

The filling material of the oral pouched nicotine product described herein may further comprise a flavouring agent. The terms *"flavour" or "flavouring agent"* are used herein for substances used to influence the aroma and/or taste of the oral pouched product. The flavours may be any food-grade natural or synthetic flavour as known in the art and may include, without limitation, essential oils, single flavour compounds, compounded flavourings, and extracts.

The filling material may comprise one, two or more flavouring agents. For example, the flavouring agent may be a non-encapsulated agent. Additionally, or alternatively, the flavouring agent may be encapsulated. The non-encapsulated flavouring agent and the encapsulated flavouring agent may be the same or different. As used herein, an encapsulated flavouring agent is a flavouring agent contained within a capsule. Accordingly, a non-encapsuled flavouring agent is not contained within a capsule.

The flavouring agent of the filling material in the oral pouched nicotine product as disclosed herein may be a hydrophobic flavouring agent.

The flavouring agent of the filling material of the oral pouched nicotine product described herein may be an oil, a liquid, a lyophilized material, a spray-dried material, or a mixture thereof. In an example, the flavouring agent(s) is/are an oil and/or a liquid.

The filling material of the oral pouched nicotine product described herein may comprise within the range of from about 0.1 wt.-% to about 10 wt.-%, such as from about 0.5 wt.-% to about 5 wt.-% of the flavouring agent, based on the total weight of the filling material.

The filling material of the oral pouched nicotine product described may comprise a humectant such as glycerol and or polypropylene glycol.

The moisture content in the filling material of the oral pouched nicotine product described herein may be present in an amount within the range of from about 0.5 wt.-% to about 60 wt.-%, such as from 4 wt.-% to about 55 wt.-%, such as from 4 wt.-% to about 50 wt.-%, such as from 4 wt.-% to about 45 wt.-%, such as from 4 wt.-% to about 40 wt.-%, based on the total weight of the filling material.

The amount of water in the filling material of the oral pouched nicotine product may be present in an amount from about 1 wt.-% or more, such as from about 2 wt.-% or more, such as from about 3 wt.-% or more, such as from about 4 wt.-% or more, such as from about 5 wt.-% or more, such as from about 6 wt.-% or more, such as from about 7 wt.-% or more, such as from about 8 wt.-% or more, such as from about 9 wt.-% or more, such as from about 10 wt.-% or more, such as from about 11 wt.-% or more, such as from about 12 wt.-% or more, such as from about 13 wt.-% or more, such as from about 14 wt.-% or more, such as from about 15 wt.-% or more, such as from about 16 wt.-% or more, such as from about 17 wt.-% or more, such as from about 18 wt.-% or more, such as from about 19 wt.-% or more, based on the total weight of the filling material in the oral pouched nicotine product.

The amount of water in the filling material of the oral pouched nicotine product may be present in an amount from 60 wt.-% or less, such as 59 wt.-% or less, such as 58 wt.-% or less, such as 57 wt.-% or less, such as 56 wt.-% or less, such as 55 wt.-% or less, such as 54 wt.-% or less, such as 53 wt.-% or less, such as 52 wt.-% or less, such as 51 wt.-% or less, such as 50 wt.-% or less, such as 49 wt.-% or less, such as 48 wt.-% or less, such as 47 wt.-% or less, such as 46 wt.-% or less, such as 45 wt.-% or less, such as 44 wt.-% or less, such as 43 wt.-% or less, such as 42 wt.-% or less, such as 41 wt.-% or less, such as 40 wt.-% or less, based on the total weight of the filling material in the oral pouched nicotine product.

The moisture content of the filling material of the oral pouched nicotine product described herein may be within the range of from about 20 wt.-% to 60 wt.-%, such as from 20 wt.-% to 55 wt.-%, such as from 20 wt.-% to 50 wt.-%, such as from 20 wt.-% to 45 wt.-%, based on the total weight of the filling material. When the amount of moisture is within the range of from about 20 wt.-% to about 45 wt.-% as described herein, the oral pouched nicotine product may be considered moist, i.e. a moist oral pouched nicotine product.

Alternatively, the moisture of the filling material of the oral pouched nicotine product described herein may be within the range of from about 0.5 wt.-% to about 20 wt.-%, such as from about 0.5 wt.-% to about 15 wt.-% , such as from about 0.5 wt.-% to about 12 wt.-%, such as from about 0.5 wt.-% to about 5 wt.-%, such as from about 0.5 wt.-% to about 3 wt.-%, such as about 3 wt.-%, based on the total weight of the filling material. When the amount of moisture is within the range of from about 0.5 wt.-% to about 12 wt.-% or from about 0.5 wt.-% to about 3 wt.-% as described herein the oral pouched nicotine product may be considered dry, i.e. a dry oral pouched nicotine product.

The oral pouched nicotine products are typically prepared as follows:
Dry ingredients are weighed and mixed in a mixer of the type Diosna for 180 s with the following settings: mixer at 180 rpm and chopper at 0 rpm
Nicotine, Tartaric acid, glycerol and water are added to the dry mix and mixed for 270 s with the following settings: mixer at 330 rpm and chopper at 900 rpm
Flavouring agents are added and mixed for 240 s with the following settings: mixer at 410 rpm and chopper at 1800 rpm
The filling material is packed in a saliva permeable pouch material (as described herein) and thereafter sealed and cut to form pouches. The moisture content of the final pouched product is adjusted by adding water if required and thereafter the oral pouched nicotine products are distributed into cans appropriate for this type of pouched product, and the cans are sealed.

### DESCRIPTION OF TEST METHODS

### Method for determining moisture content, Loss on Drying (LOD)

The moisture content as referred to herein may be determined by using the method as described in *Moisture Content in Tobacco, Tobacco Products, Tobacco Derived Products and Fiber Based Matrices by Loss on Drying according to Coresta*/*CDC*

In this method moisture content/dry content is determined gravimetrically by a given amount of the sample being collected and the weight measured before evaporation of the moisture in an oven for 3 hours at 99±1°C and the drying process is complete. The method is used for quantitative analysis of moisture in the range of 1% to 60%.

### Method for determining pH

The pH of the filling material is determined by using the method *Measurement of pH in Tobacco, Tobacco products, Fibre-based Matrices and Tobacco Derived Products with pH Electrode, Coresta recommended method No. 69.*

### EXAMPLES

### Example 1: Effects of stearates on the pH-stability of pouched nicotine products during storage?

Materials used in the filling material in the oral pouched nicotine products in Table 1 below:
Nicotine: CAS No. 54-11-5
Sodium Carbonate, Na₂CO₃, CAS No. 497-19-8
Microcristalline cellulose (MCC), CAS No. 9004-34-6
Sodium stearate: C₁₈H₃₅NaO₂, CAS No. 822-16-2
Magnesium Stearate: [CH₃(CH₂)₁₆CO₂]₂Mg, CAS No. 557-04-0
Calcium Stearate: [CH₃(CH₂)₁₆COO]₂Ca, CAS No. 1592-23-0
NaCl: CAS No. 7647-14-5
Acesulfame potassium: CAS No. 55589-62-3
Glycerol: CAS No. 56-81-5
Tartaric Acid: CAS No. 87-69-4
Flavouring agent: "Spearmint"
Water: tap water

**Table 1**

| **Ingredient (wt.-%)** | **Ref S2** | **Na St S2** | **Mg St S2** | **Ca St S2** | **Ref S4** | **Na St S4** | **Mg St S4** | **Ca St S4** |
|---|---|---|---|---|---|---|---|---|
| **Nicotine** | 0.9 | 0.9 | 0.9 | 0.9 | 1.6 | 1.6 | 1.6 | 1.6 |
| **Na₂CO₃** | 0.4 | 0.4 | 0.4 | 0.4 | 0.8 | 0.7 | 0.7 | 0.7 |
| **MCC** | 40.2 | 39.8 | 39.8 | 39.8 | 38.8 | 38.2 | 38.2 | 38.2 |
| **NaCl** | 3.5 | 3.5 | 3.5 | 3.5 | 3.5 | 3.5 | 3.5 | 3.5 |
| **Acesulfame potassium** | 0.4 | 0.4 | 0.4 | 0.4 | 0.4 | 0.4 | 0.4 | 0.4 |
| **Glycerol** | 5.8 | 5.8 | 5.8 | 5.8 | 5.8 | 5.8 | 5.8 | 5.8 |
| **Tartaric acid** | 0.5 | 0.5 | 0.5 | 0.5 | 0.8 | 0.8 | 0.8 | 0.8 |
| **Na Stearate** | 0 | 0.4 | 0 | 0 | 0 | 0.7 | 0 | 0 |
| **Mg Stearate** | 0 | 0 | 0.4 | 0 | 0 | 0 | 0.7 | 0 |
| **Ca Stearate** | 0 | 0 | 0 | 0.4 | 0 | 0 | 0 | 0.7 |
| **Flavouring agent** | 0.3 | 0.3 | 0.3 | 0.3 | 0.3 | 0.3 | 0.3 | 0.3 |
| **Water** | 48 | 48 | 48 | 48 | 48 | 48 | 48 | 48 |
| **Total** | 100 | 100 | 100 | 100 | 100 | 100 | 100 | 100 |

Pouches containing filling material as listed in Table 1 were manufactured as follows:
All dry ingredients were weighed and mixed mixer of the type Diosna for 180 s with the following settings: mixer at 180 rpm and chopper at 0 rpm.
Nicotine, tartaric acid, glycerol and water were added to the dry mix and mixed for 270 s with the following settings: mixer at 330 rpm and chopper at 900 rpm.
Flavouring agents were added and mixed for 240 s with the following settings: mixer at 410 rpm and chopper at 1800 rpm
Saliva-permeable pouch material (as described herein) was filled with respective filling material and thereafter sealed and cut to form pouches. Pouches were moisturized to have approximate moisture content of 48 wt.-%. The moisturized pouches were canned in cans with a diameter of 70 mm and made from polypropylene with twenty-one pouches per can. The lids and bottom parts of the cans were sealed together by means of adhesive banderol extending around the outer perimeter of the can, and thereafter the sealed cans were stored under the conditions described below.

One hundred and thirty cans were prepared for each filling composition. Half of the cans were stored in Zone II at 25°C with a relative humidity (RH) of 60%, and the other half in Zone IV at 30°C with a relative humidity (RH) of 75%. Nine cans from each filling composition and zone were tested at 0, 2, 4, 6, 16, 24 and 32 weeks in accordance with the methods described in the section Description of Test Methods above.

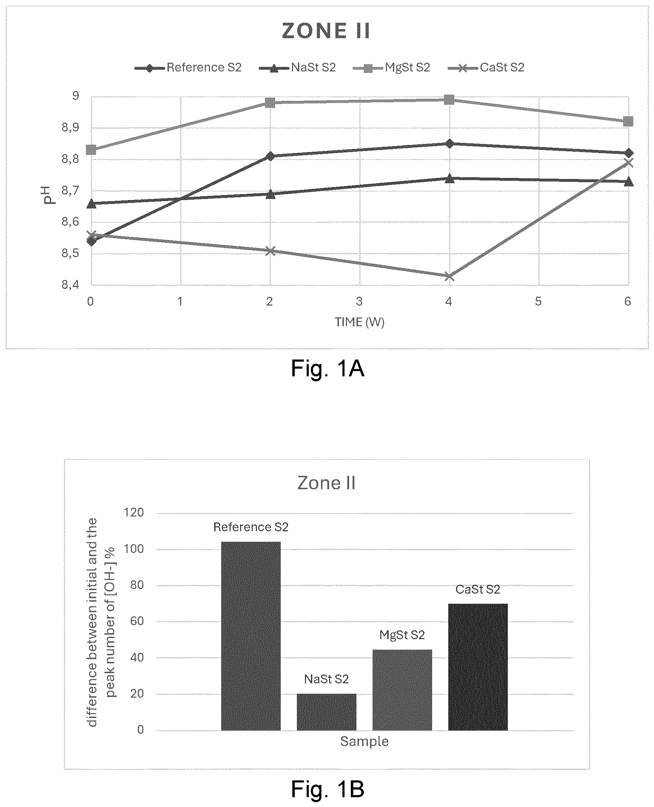

Figures 1-4 disclose the change in pH/concentration of OH⁻ during storage of the different pouched filling compositions over time in the different zone conditions II or IV.

Figures 1A and 1B show oral pouched nicotine products containing 0.9 wt.-% of nicotine, without any stearate (Ref S2), with sodium stearate (NaSt S2), with magnesium stearate (MgSt S2) or calcium stearate (CaSt S2) and stored in Zone II. Fig. 1A discloses that the pH of oral pouched nicotine products containing 0.9 wt.-% of nicotine and sodium stearate remain more stable over six weeks compared to filling material without any stearate when stored in Zone II. Surprisingly, it turns out that sodium stearate has a more stabilizing effect on pH of the filling material than magnesium stearate and calcium stearate have.

The stabilizing effect of sodium stearate on filling materials compared to filling materials without stearate or filling materials with magnesium stearate or calcium stearate is clearly seen in Fig. 1B wherein the variation in [OH-] ions between week 0 and the peak number of [OH-] ions during storage is plotted as a bar chart. Fig. 1B discloses that the concentration of [OH-] ions in oral pouched nicotine products without stearate varies more than 100% during storage, i.e., the concentration of hydroxide ions more than doubles at one point during the six weeks of storage, while the concentration of hydroxide ions in filling material that includes sodium stearate varies with less than 20%. Filling materials including magnesium stearate or calcium stearate vary with about 43% and 70 % respectively.

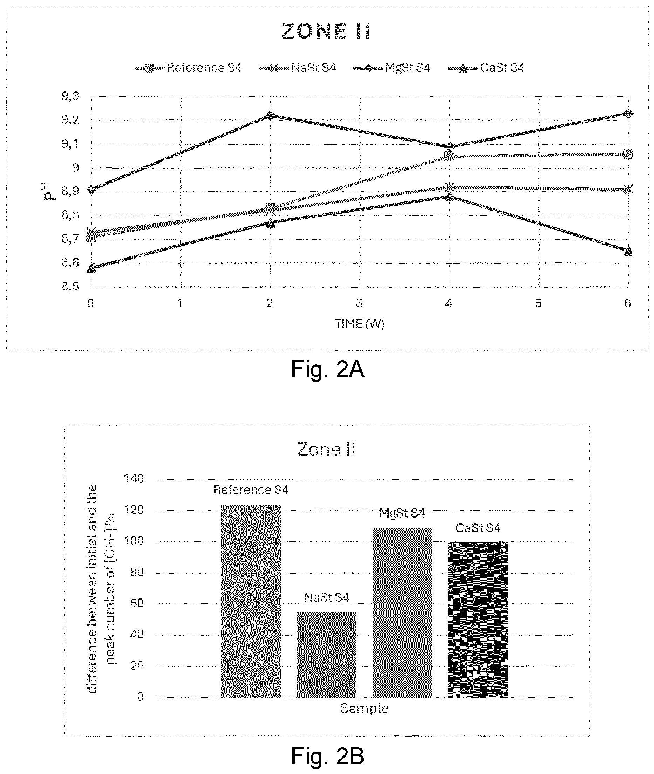

Figures 2A and 2B show oral pouched nicotine products containing 1.6 wt.-% of nicotine, without any stearate (Ref S4), with sodium stearate (NaSt S4), with magnesium stearate (MgSt S4) or calcium stearate (CaSt S4) and stored in Zone II. The graph in fig. 2A discloses that the pH of oral pouched nicotine products containing 1.6 wt.-% of of nicotine and sodium stearate remain more stable over six weeks compared to filling material without stearate when stored in Zone IV. Also, for higher concentrations of nicotine, sodium stearate has a more stabilizing effect on the pH of the filling material than magnesium stearate and calcium stearate have.

The bar chart in Fig. 2B discloses that the concentration of [OH-] ions in oral pouched nicotine products without stearate varies with more than 120% during storage, while pH in filling material with sodium stearate only varies with about 55%. Filling materials which include magnesium stearate or calcium stearate vary with about 110% and 100 % respectively.

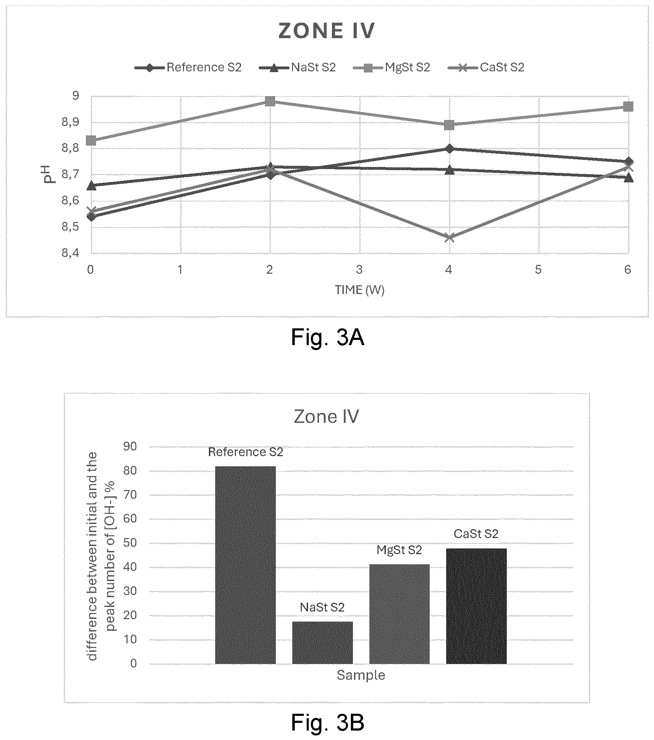

Figures 3A and 3B show oral pouched nicotine products containing 0.9 wt.-% of nicotine, without any stearate (Ref S2), with sodium stearate (NaSt S2), with magnesium stearate (MgSt S2) or calcium stearate (CaSt S2) that are stored in Zone IV. The graph in fig. 3A discloses that the pH of oral pouched nicotine products containing 0.9 wt.-% of of nicotine and sodium stearate remain more stable over six weeks compared to filling material without stearate when stored in Zone IV. Magnesium stearate and calcium stearate have a less stabilizing effect on pH than sodium stearate.

The bar chart in Fig. 3B discloses that the concentration of [OH-] ions in oral pouched nicotine products without stearate varies with about 80% during storage in zone IV, while pH in filling material that includes sodium stearate only varies with about 15% in the same storage conditions. Filling materials comprising magnesium stearate or calcium stearate vary with about 40% and 45 % respectively.

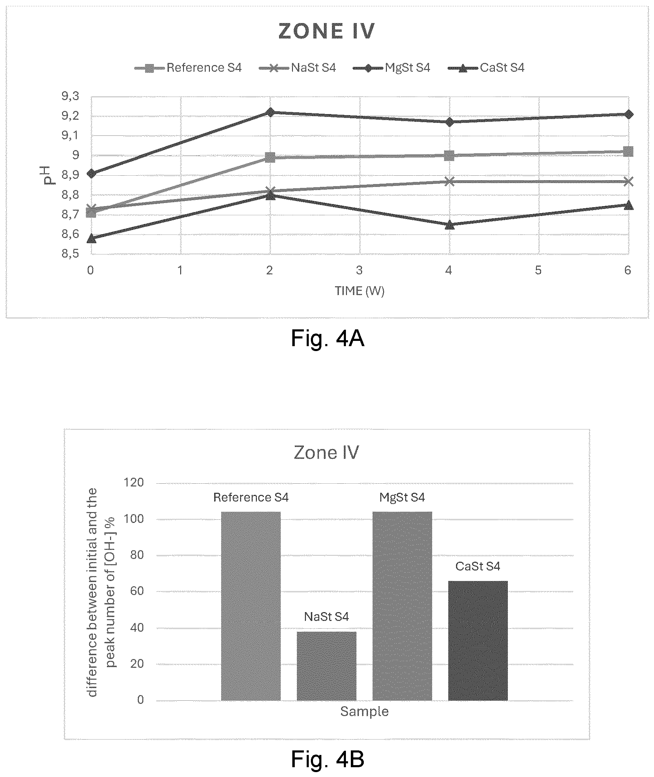

Figures 4A and 4B show oral pouched nicotine products containing 1.6 wt.-% of nicotine, without any stearate (Ref S4), with sodium stearate (NaSt S4), with magnesium stearate (MgSt S4) or calcium stearate (CaSt S4) that have been stored in Zone IV. The graph in fig. 4A discloses that the pH of oral pouched nicotine products containing 1.6 wt.-% of of nicotine and sodium stearate remain more stable over six weeks compared to filling material without stearate when stored in Zone IV. Also, for higher concentrations of nicotine, sodium stearate has a more stabilizing effect on the pH of the filling material than magnesium stearate and calcium stearate have.

The bar chart. Fig. 4B discloses that the concentration of [OH-] ions in oral pouched nicotine products without stearate varies with about 100% during storage, while pH in the filling material with sodium stearate only varies with about 38%. Filling materials comprising magnesium stearate or calcium stearate vary with about 100% and 65 % respectively.

It can be concluded that oral pouched nicotine products that contain carbonate as the only pH regulating agent have a filling material with a pH that fluctuates considerably during storage of the product. However, addition of stearate to the filling material improves its pH stability during storage. This is especially true for filling materials with lower concentrations of nicotine. It is also evident that sodium stearate provides better stabilizing effect on the pH of the filling material than magnesium stearate and calcium stearate do. This stabilizing effect of sodium stearate is seen in all filling materials but is especially obvious in filling materials with higher concentrations of nicotine as seen in e.g., Figs. 2 and 4.

### Example 2: How does the concentration of Na-stearate in the filling composition affect the pH stability during storage of pouched nicotine products?

Filling compositions containing 10 wt.-% in S2 samples (i.e., filling compositions containing 0.9 wt.-% of nicotine per pouch), and 20 wt.-% of sodium stearate in S4 samples i.e., filling compositions containing 1.6 wt.-% of of nicotine per pouch) were prepared and pouched as described in Example 1 above. Since sodium stearate acts as a weak base, the amount of pH adjusting agent Na₂CO₃ was decreased with 0.02 wt.-% to maintain the initial pH of the pouched product within pH 8.5-8.8.

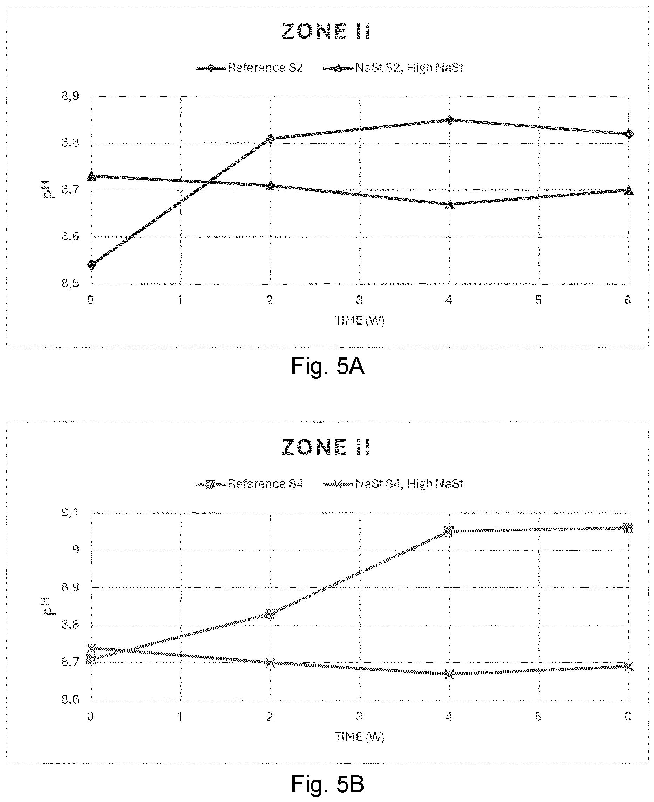

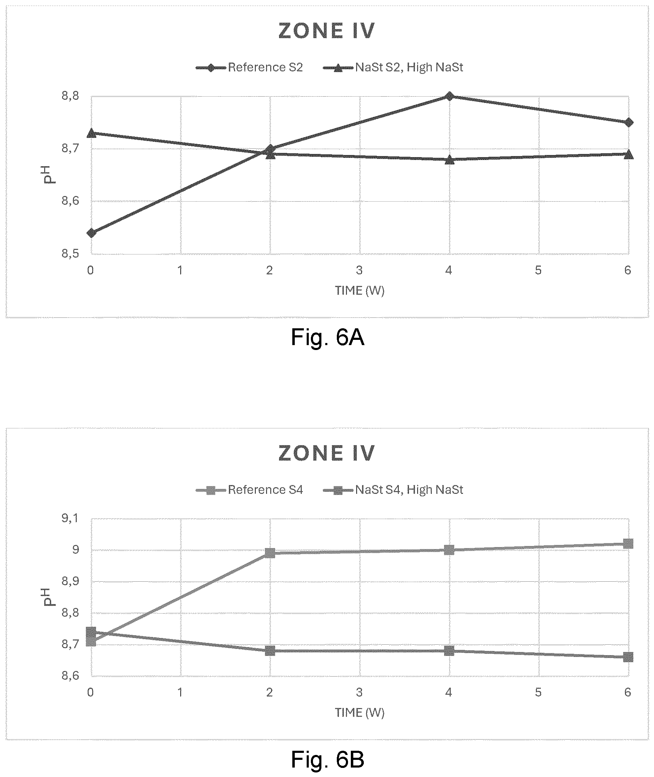

Figures 5 and 6 disclose that high concentrations of sodium stearate in the filling material of oral pouched nicotine products have a substantial effect on the stability of pH both at low and high concentrations of nicotine and regardless of storage conditions.

## Claims

1. An oral pouched nicotine product comprising a filling material and a saliva-permeable pouch enclosing the filling material, the filling material comprising
- a nicotine compound;
- a pH-regulating agent; and
- a monovalent metal salt of a stearic acid.

2. The oral pouched nicotine product according to claim 1, wherein the monovalent metal salt of a stearic acid is selected from the group consisting of sodium stearate, potassium stearate and a combination thereof.

3. The oral pouched nicotine product according to claim 1, wherein the monovalent metal salt of a stearic acid is sodium stearate.

4. The oral pouched nicotine product according to any one of the preceding claims, wherein the monovalent metal salt of a stearic acid is present in an amount of less than 20 wt.-% based on the total weight of the filling material, such as from about 0.1 wt.-% to about 20 wt.-% based on the total weight of the filling material.

5. The oral pouched nicotine product according to any one of the preceding claims, wherein the pH regulating agent is present in an amount within the range of from about 0.02 wt.-% to about 20 wt.-%, such as from about 0.04 wt.-% to about 0.15 wt.-% or from about 2 wt.-% to about 5 wt.-%, or from about 6 wt.-% to about 10 wt.-%, or from about 11 wt.-% to about 15 wt.-%, or from about 12 wt.-% to about 20 wt.-% based on the total weight of the filling material.

6. The oral pouched nicotine product according to any one of the preceding claims, wherein the pH-regulating agent is a carbonate or a bicarbonate.

7. The oral pouched nicotine product according to claim 5, wherein the pH regulating agent is selected from the group consisting of sodium carbonate, potassium carbonate, sodium bicarbonate or a potassium bicarbonate and any combination thereof.

8. The oral pouched nicotine product according to any one of the preceding claims, wherein the filling material does not comprise a tobacco material.

9. The oral pouched nicotine product according to any one of the preceding claims, wherein the filling material further comprises a tobacco material in an amount within the range of from about 0.1 wt.-% to about 5 wt.-%, such as from about 0.1 wt.-% to about 10 wt.-%, such as from about 0.1 wt.-% to about 5 wt.-%, such as from about 0.1 wt.-% to about 1 wt.-%, based on the total weight of the filling material.

10. The oral pouched nicotine product according to any one of the preceding claims, wherein the pouched nicotine product has a moisture content in the range of from about 1.0 wt.-% to about 60 wt.-%, such as from about 20 wt.-% to about 60 wt.-%, such as from about 1 wt.-% to about 20 wt.-%, based on the total weight of the filling material.

11. The oral pouched nicotine product according to any one of the preceding claims, wherein said filling material of said oral pouched nicotine product has a pH from about 5 to about 10, such as from about 7 to about 9.5, such as from about 8 to about 9, when the filling material is dispersed in water.

12. The oral pouched nicotine product according to any one of the preceding claims, wherein at least part of the filling material is water-insoluble.

13. The oral pouched nicotine product according to any one of the preceding claims, wherein the filling material comprises one or more of maize fibers, oat fibers, tomato fibers, barley fibers, rye fibers, sugar beet fibers, buck wheat fibers, wheat fibers, pea fibers, potato fibers, apple fibers, cocoa fibers, bamboo fibers, and citrus fibers.

14. The oral pouched nicotine product according to any one of the preceding claims, wherein the filling material comprises microcrystalline cellulose.

15. The oral pouched nicotine product according to any one of the preceding claims, wherein the nicotine compound is selected from the group consisting of nicotine hydrochloride, nicotine dihydrochloride, nicotine monotartrate, nicotine bitartrate, nicotine bitartrate dihydrate, nicotine sulphate, nicotine zinc chloride monohydrate, nicotine salicylate, nicotine polacrilex and any combination thereof.

16. The oral pouched nicotine product according to claim 15, wherein the nicotine compound is one or more of nicotine bitartrate and nicotine bitartrate di hydrate.

17. The oral pouched nicotine product according to any one of the preceding claims, wherein pH of the filling material of the oral pouched nicotine product does not exceed 9.5 upon storage and/or changes by no more than plus or minus 0.5 pH units upon storage, such as storage taking place at a relative humidity from 60 percent to 75 percent, at a temperature from 22 degrees centigrade to 30 degrees centigrade, and/or for a time of 15 weeks.

18. A packaging container comprising a plurality of oral pouched nicotine products according to anyone of the preceding claims.

19. A method for manufacturing a filling material as defined in any one of claims 1-17
